# EUROPÄISCHE PATENTANMELDUNG

(11) **EP 3 047 840 A1**
(43) Veröffentlichungstag der Anmeldung: **27.07.2016**
(21) Anmeldenummer: 15151941.0
(22) Anmeldetag: 21.01.2015
(51) Int. Cl.: A61K 8/25, A61Q 5/02, A61Q 5/12, A61K 8/73, A61K 8/11

(54) **Pulverförmige Zubereitung enthaltend Partikel, die Tröpfchen einer wässerigen Phase umhüllen, und eine versprühbare Dispersion hiervon**

(71) Anmelder: Evonik Degussa GmbH, 45128 Essen (DE)
(72) Erfinder: Riedemann, Heike, 63776 Mömbris (DE); Münzenberg, Dr., Jörg, 63456 Hanau (DE); Winter, Patrick, 45473 Mülheim an der Ruhr (DE); Schwab, Dr., Peter, 45133 Essen (DE)

(57) **Zusammenfassung**

Pulverförmige Zubereitung enthaltend Tröpfchen einer wässerige Phase umhüllende Partikel, wobei die Zubereitung hydrophobe Siliciumdioxidpartikel und eine hydrophobierte Stärke enthält, wenigstens ein Teil der hydrophoben Siliciumdioxidpartikel Bestandteil der die Tröpfchen der wässerigen Phase umgebenden Hülle bildet.

Dispersion enthaltend die pulverförmige Zubereitung, die pulverförmige Zubereitung , ein Silikonöl und/oder ein organomodifiziertes Siloxan, wobei das organomodifizierte Siloxan aus wenigstens einem Mitglied der Gruppe bestehend aus einem Silikonpolyether, einem kationischen Polysiloxan und einem mindestens eine Aminogruppe aufweisenden Polysiloxan ausgewählt ist, einen Alkohol und ein Treibmittel.

## Beschreibung

Die Erfindung betrifft eine pulverförmige Zubereitung enthaltend Partikel, die Tröpfchen einer wässerigen Phase umhüllen. Die Erfindung betrifft weiterhin eine versprühbare Dispersion und deren Verwendung als Trockenshampoo.

Trockenshampoo-Zubereitungen sind im Stand der Technik bekannt. Sie werden gewöhnlich auf das Haar aufgesprüht, ein Lösungsmittel in der Trockenshampoo-Zubereitung verdunstet und es verbleibt ein Pulver, welches Talg aus dem Haar aufnimmt. Das so beladene Pulver wird schließlich, zumindest teilweise, ausgekämmt.

Die Trockenshampoo-Zubereitung enthält in der Regel modifizierte Stärken zum Aufnehmen des Talges aus den Haaren. So werden mittels Octenylbernsteinsäureanhydrid (OSA) modifizierte und mit Aluminiumsulfat behandelte Stärken eingesetzt. Ebenso sind mittels des Anhydrides der Dodecenylbernsteinsäure modifizierte, auf Calcium basierende Stärkederivate bekannt.

In WO2012/104362 wird eine mit Silikat oder Siliconat modifizierte Stärke, als Bestandteil eines Trockenshampoos offenbart. Das Trockenshampoo kann die Stärke in einer Menge von etwa 0,1 bis etwa 99 Gew.-%, bezogen auf das Gesamtgewicht des Trockenshampoos enthalten. Zusätzlich zur Stärke kann es ein Lösungsmittel und Treibmittel enthalten.

In WO2011/056625 wird eine Trockenshampoo-Zubereitung offenbart, die 1 - 35% eines Trägermaterials, 3 - 5% einer mittels OSA modifizierte und mit Aluminiumsulfat behandelte Stärke, 2 - 4% eines Materiales aus Ton und 75 - 80% eines Treibmittels enthält.

In WO2014/004847 wird ein Trockenshampoo offenbart, welches 5 - 12% partikelförmige, hydrophil wie hydrophob modifizierte, Tapiokastärke, 30 - 50% Alkohol, 0,1% - 0,3% Redispergierungsmittel und 40 - 60% Treibmittel und weniger als 1% nicht flüchtige Öle enthält. Das Redispergierhilfsmittel dient dazu, zu verhindern, dass sich die Stärke-Partikel, die sich zwischen zwei Anwendungen absetzen, zu größeren Aggregaten vereinigen, die durch Schütteln nicht mehr ausreichend aufgebrochen werden können. Als Redispergierungsmittel kommen pyrogene Kieselsäuren in Frage, hydrophile wie auch hydrophobe Typen.

Den im Stand der Technik genannten Formulierungen ist gemein, dass die Kämmbarkeit, der Weichgriff und die antistatischen Eigenschaften verbesserungswürdig sind.

Gegenstand der Erfindung ist eine pulverförmige Zubereitung enthaltend Partikel, die Tröpfchen einer wässerigen Phase umhüllen, wobei die Zubereitung hydrophobe Siliciumdioxidpartikel und eine hydrophobierte Stärke enthält und wenigstens ein Teil der hydrophoben Siliciumdioxidpartikel Bestandteil der die Tröpfchen der wässerigen Phase umgebenden Hülle bildet.

Der Erfindungsgegenstand stellt eine besondere Form von so genanntem "trockenen Wasser" ("Dry Water") dar, bei dem Flüssigkeitströpfchen von hydrophoben Siliciumdioxidpartikeln umhüllt werden. Durch die Hülle werden die Flüssigkeitströpfchen an der Koaleszenz gehindert, und es entsteht ein pulverförmiges Gemisch. Durch Reibung oder Druck kann die Hülle aufgebrochen werden, so dass die wässerige Phase freigesetzt wird und für den gewünschten Verwendungszweck zur Verfügung steht.

Bei der erfindungsgemäßen pulverförmigen Zubereitung kann die Hülle als weiteren Bestandteil wenigstens einen Teil der hydrophobierten Stärke enthalten.

Das Gewichtsverhältnis hydrophobe Siliciumdioxidpartikel zu hydrophobierter Stärke der pulverförmigen Zubereitung ist bevorzugt 1:50 bis 1:2.

Weiterhin ist bevorzugt, dass das Gewichtsverhältnis Summe hydrophobe Siliciumdioxidpartikel und hydrophobierte Stärke zur wässerigen Phase 80:20 bis 20:80, bevorzugt 70:30 bis 40:60, ist.

Bei der wässerigen Phase kann sich um eine Lösung, eine Emulsion oder eine Dispersion handeln. Bevorzugt ist die wässerige Phase eine Lösung. Bevorzugt ist deren Anteil an Wasser mindestens 90 Gew.-%, besonders bevorzugt mindestens 95 Gew.-% und ganz besonders bevorzugt mindestens 99 Gew.-%. Es ist auch möglich, dass die wässerige Phase nur aus Wasser besteht.

Der Anteil an gelösten, emulgierten oder dispergierten Stoffen ist so ausgewählt, dass sie die Oberflächenspannung nicht oder nur unwesentlich reduzieren. Bevorzugt enthält die wässerige Phase der pulverförmigen Zubereitung weniger als jeweils 0,3 Gew.-% eines Silikonöles und/oder eines organomodifizierten Siloxanes. Organomodifizierte Siloxane umfassen Silikonpolyether, kationische Polysiloxane und mindestens eine Aminogruppe aufweisende Polysiloxane. Besonders bevorzugt enthält sie weniger als 0,1 Gew.-%. In einer besonderen Ausführungsform ist sie frei von Silikonöl und organomodifizierten Siloxanen.

Ein weiterer Gegenstand der Erfindung ist ein Verfahren zur Herstellung der pulverförmigen Zubereitung, bei dem die Bestandteile der flüssigen Phase zunächst vorgelegt und gegebenenfalls homogenisiert werden, anschließend die hydrophoben Siliciumdioxidpartikel und die hydrophobierte Stärke zugegeben werden und die Mischung geschert wird. Die Scherung muss ausreichend hoch für die Bildung der pulverförmigen Zubereitung sein, jedoch nicht so hoch, dass die einmal gebildete pulverförmigen Zubereitung unter Verlust der Umhüllung in eine Dispersion oder Creme verwandelt wird. Dispergieraggregate zur Herstellung von "trockenem Wasser" sind hinreichend beschrieben.

Bevorzugt werden hydrophobe, pyrogen hergestellte Siliciumdioxidpartikel eingesetzt. Sie werden durch Flammenhydrolyse von flüchtigen Siliciumverbindungen wie organischen und anorganischen Chlorsilanen hergestellt. Bei diesem Verfahren wird ein verdampftes oder gasförmiges, hydrolysierbares Siliciumhalogenid mit einer Flamme zur Reaktion gebracht, die durch Verbrennung von Wasserstoff und eines sauerstoffhaltigen Gases gebildet worden ist. Die Verbrennungsflamme stellt dabei Wasser für die Hydrolyse des Siliciumhalogenides und genügend Wärme zur Hydrolysereaktion zur Verfügung. So hergestellte Siliciumdioxidpartikel werden als pyrogene Kieselsäure bezeichnet. Die so hergestellte Kieselsäure ist eine hydrophile Kieselsäure, die in einem nachfolgenden Schritt hydrophobiert wird.

Bei den hydrophoben Siliciumdioxidpartikeln handelt es sich bevorzugt um silanisierte Siliciumdioxidpartikel. Zur Silanisierung können beispielsweise Halogenorganosilane, Alkoxysilane, Silazane und/oder Siloxane eingesetzt werden. Besonders bevorzugte Silanisierungsmittel sind Trimethoxyoctylsilan, Hexamethyldisilazan und Octamethylcyclotetrasiloxan.

Die spezifische Oberfläche der hydrophoben Siliciumdioxidpartikel ist nicht beschränkt.

Bevorzugt enthält die erfindungsgemäße pulverförmige Zubereitung solche Siliciumdioxidpartikel mit einer spezifischen Oberfläche von 30 bis 500 m²/g, besonders bevorzugt 50 bis 400 m²/g und ganz besonders bevorzugt solche von 80 bis 290 m²/g. Die BET-Oberfläche wird nach DIN 66131 bestimmt.

Die Methanolbenetzbarkeit der hydrophoben Siliciumdioxidpartikel ist bevorzugt wenigstens 30 Vol.-% Methanol, besonders bevorzugt 50 - 70 Vol.-% Methanol. Bei der Bestimmung der Methanolbenetzbarkeit werden in transparente Zentrifugenröhrchen jeweils 0,2 g ± 0,005 g der Siliciumdioxidpartikel eingewogen. Es werden zu jeder Einwaage 8,0 ml eines Methanol/Wasser Gemisches mit jeweils 10, 20, 30, 40, 50, 60, 70 und 80 Vol.-% Methanol zugegeben. Die verschlossenen Röhrchen werden 30 Sekunden geschüttelt und anschließend 5 Minuten bei 2500 U/min zentrifugiert. Die Sedimentvolumina werden abgelesen, in Prozent umgerechnet und graphisch gegen den Methanolgehalt in Vol.-% aufgetragen. Der Wendepunkt der Kurve entspricht der Methanolbenetzbarkeit.

Beispiele für kommerziell erhältliche hydrophobe Siliciumdioxidpartikel sind beispielsweise AEROSIL^{®} R 104 (ca. 150 m²/g; 55 Vol.-% Methanol); AEROSIL^{®} R 106 (ca. 250 m²/g; 50 Vol.-% Methanol), AEROSIL^{®} R 202 (ca. 100 m²/g; 75 Vol.-% Methanol), AEROSIL^{®} R 805 (ca. 150 m²/g; 60 Vol.-% Methanol), AEROSIL^{®} R 812 (ca. 260 m²/g; 60 Vol.-% Methanol), AEROSIL^{®}R 812 S (ca. 220 m²/g; 65 Vol.-% Methanol), AEROSIL^{®} R 8200 (ca. 160 m²/g; 65 Vol.-% Methanol), alle von Evonik Industries. Die Angaben in Klammern beziehen sich auf die spezifische BET-Oberfläche und die Methanolbenetzbarkeit.

Als hydrophobierte Stärke wird bevorzugt eine alkyl- oder alkenylmodifizierte Stärke eingesetzt. Besonders bevorzugt kann dies eine mit Octenylbernsteinsäureanhydrid oder Dodecenylbernsteinsäure modifizierte Stärke sein, die jeweils mit einem Salz des Aluminiums oder Calciums behandelt ist. Besonders bevorzugt ist eine mit Octenylbernsteinsäureanhydrid und mit einem Salz des Aluminiums behandelte Stärke. Die zugrundeliegende Stärke wird bevorzugt ausgewählt aus der Pflanzenstärke von Mais, Weizen, Reis, Tapioka, Kartoffel, Sago, Erbse und Süßkartoffel. Kommerziell erhältliche, geeignete Stärken sind beispielsweise DRY-FLO^{®} PC starch (INCI Aluminum Starch Octenylsuccinate), NATRASORB^{®} HFB starch, (INCI Aluminum Starch Octenylsuccinate (and) Acrylates Copolymer (and) Magnesium Carbonate) und DRY-FLO^{®} TS (INCI Tapioca Starch Polymethylsilsesquioxane), alle Akzo Nobel. Es können ebenso Mischungen hydrophobierter Stärken vorliegen.

Ein weiterer Gegenstand der Erfindung ist eine Dispersion enthaltend
die erfindungsgemäße pulverförmige Zubereitung,
ein Silikonöl und/oder ein organomodifiziertes Siloxan, wobei das organomodifizierte Siloxan aus wenigstens einem Mitglied der Gruppe bestehend aus einem Silikonpolyether, einem kationischen Polysiloxan und einem mindestens eine Aminogruppe aufweisenden Polysiloxan ausgewählt ist,
einen Alkohol und ein Treibmittel.

In einer besonderen Ausführungsform enthält die Dispersion
5 bis 30 Gew.-%, besonders bevorzugt 15 bis 25 Gew.-%, pulverförmige Zubereitung,
0,1 bis 5 Gew.-%, besonders bevorzugt 0,5 bis 3 Gew.-%, eines Silikonöles und/oder eines organomodifizierten Siloxanes,
3 bis 40 Gew.-%, besonders bevorzugt 5 bis 20 Gew.-%, Alkohol und
30 bis 90 Gew.-%, besonders bevorzugt 60 bis 80 Gew.-%, Treibmittel.

Die erfindungsgemäße Dispersion ist versprühbar. Beim Versprühen der Dispersion mittels eines geeigneten Sprühsystems, beispielsweise einer Sprühdose, wird ein Aerosol erzeugt. Aerosol steht insbesondere für ein System aus Gasen mit darin verteilten Teilchen und Tröpfchen. Dabei dient ein verflüssigtes Druckgas als Treibgas. Beim Öffnen des Druckventils entweicht die Dispersion durch eine Düse und das Treibmittel verdampft. Wird die Zubereitung als Trockenshampoo eingesetzt, so wird beim Auftreffen der pulverförmigen Zubereitung auf den Haaren bzw. beim Verreiben der Dispersion auf den Haaren die wässerige Phase der pulverförmigen Zubereitung freigesetzt.

Bei den Silikonölen kann es sich um lineare oder verzweigte Polysiloxane handeln, die methyl- oder hydroxyl-verkappt sind und eine Viskosität > 100 mPas/25°C aufweisen.

Organomodifizierte Siloxane sind beispielsweise Polyethersiloxane, beispielsweise unter der Namensreihe TEGOPREN® oder der Namensreihe ABIL® kommerzialisiert.

Besonders geeignete Polyethersiloxanderivate sind solche der allgemeinen Formel I 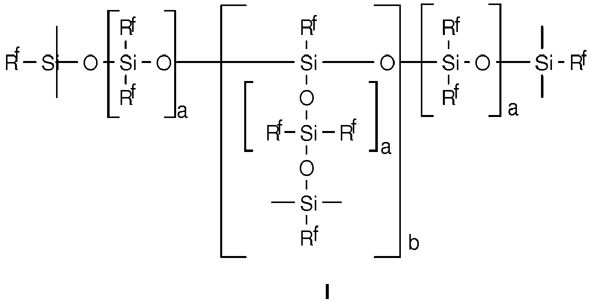 wobei der Rest
- R^{f}: der Rest R¹ sein kann, wobei
- R¹: ein Alkylrest mit 1 bis 4 C-Atomen, oder ein Arylrest ist, oder
- R^{f}: der Rest R² oder R³ ist, mit der Maßgabe, dass mindestens ein Rest R^{f} der Rest R² ist, wobei
- R² und R³: unabhängig voneinander Polyetherreste der Formel II

(F)_{q}(O(C₂H_{4-d}R'_{d}0)ₘ(CₓH₂ₓO)ᵣZ)_{w} **(II)**

sind, mit der Bedeutung
- d: 1 bis 3
- m: > 1
- q: 0 oder 1
- x: 2 bis 10
- r: > 1
- w: 1 bis 4
- F: ein Kohlenwasserstoffrest, der auch verzweigt sein kann,
- R': ein Wasserstoffrest, ein einwertiger Kohlenwasserstoffrest mit 1 bis 18 C-Atomen
- Z: ein H-Atom oder ein einwertiger organischer Rest wie Alkyl oder Alkyl- oder Arylester ist,
und wobei in Formel (**I**)
- b: eine Zahl von 0 bis 8 ist,
- a: eine Zahl von 1 bis 100 ist, wenn b eine Zahl von 6 bis 8 ist,
- a: eine Zahl von 1 bis 200 ist, wenn b eine Zahl von 3 bis 6 ist,
- a: eine Zahl von 1 bis 300 ist, wenn b eine Zahl von 0 bis 3 ist.

Die Werte von a und b sind als durchschnittliche Werte zu verstehen, da die erfindungsgemäß mitverwendeten Siliconpolyether-Copolymere in Form von regelfalläquilibrierten Gemischen vorliegen.

Die Reste R¹ sind Alkylreste mit 1 bis 4 Kohlenstoffatomen, wie Methyl-, Ethyl-, Propyl- oder Butylreste, oder Arylreste, wobei die Phenylreste bevorzugt sind. Herstellungs- und preisbedingt sind die Methylreste bevorzugt, so dass mindestens 80 % der Reste R¹ Methylreste sind. Besonders bevorzugt sind solche Polysiloxane, bei denen alle Reste R¹ Methylreste sind.

Das Siloxangemisch kann geradkettig (b = 0) oder verzweigt sein (b > 0 bis 8). Der Wert von a kann erfahrungsgemäß nur in der angegebenen Weise mit Werten von b kombiniert werden, da ansonsten die erhöhte Viskosität eine Handhabung unmöglich macht.

Besonders bevorzugte Siliconpolyether-Copolymere sind solche der allgemeinen Formel III 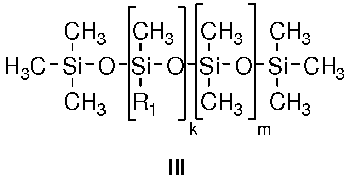 worin
- m: = 0 bis 30,
- k: = 1 bis 5,
- R¹: ein Allylalkohol oder Alkyl-gestarteter Polyether, welcher mit 1 bis 10 Ethylenoxidmolekülen und zwischen 1 und 25 Propylenoxidmolekülen umgesetzt ist,
bedeutet.

Geeignete Polyethersiloxane sind Strukturen der Formel IV 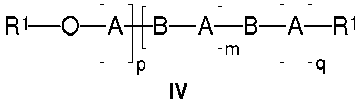 wobei der Rest
A ein Polyoxyalkylenblock der durchschnittlichen Formel V ist,

[(C₂H₄₋dR'_{d}O)ₙ(CₓH₂ₓO)ᵣ(C₂H_{4-d} R"_{d}O)ₜ] **V**

wobei
- d: 1 bis 3,
- n: ≥ 0,
- x: 2 bis 10,
- r: ≥ 0,
- t: ≥ 0,
- n: + r + t ≥ 1,
sind und
- R': ein einwertiger aromatischer gegebenenfalls substituierter Kohlenwasserstoffrest,
- R": ein Wasserstoffrest oder ein einwertiger Kohlenwasserstoffrest mit 1 bis 18 C-Atomen,
- R¹: ein H-Atom, ein einwertiger organischer linearer oder verzweigter Alkylrest mit der Kettenlänge C₁-C₄₀ oder ein Carboxyrest eines gegebenenfalls verzweigten Alkyl- oder Arylesters ist,
- B: ein Polysiloxanblock der durchschnittlichen Formel VI ist, 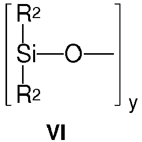
wobei
- R²: gleich oder verschieden ist und einen Alkylrest mit 1 bis 4 Kohlenstoffatomen oder einen Phenylrest umfasst und
- y: einen Wert von 5 bis 200,
- m: einen Wert von 2 bis 100,
- p: einen Wert von 0 oder 1 und
- q: einen Wert von 0 oder 1,
hat,

oder der Formel VII 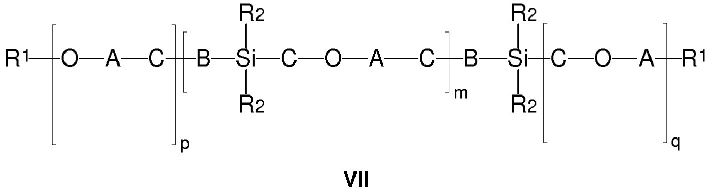 wobei die Reste
R¹, A, B und m, p und q die oben genannte Bedeutung haben und
C ein linearer oder verzweigter Alkylenrest mit 2 bis 20 Kohlenstoffatomen ist.

Die Verbindungen der angegebenen Strukturen liegen als statistische Mischungen vor, daher entsprechen die angegebenen Indexzahlen den zahlenmäßigen Mittelwerten der Mischungen.

Bei den kationischen Polysiloxanen kann es sich bevorzugt um solche der allgemeinen Formel

[Z-M-Si(CH₃)₂O-[Si(CH₃)₂O]ₙSi(CH₃)₂-M-Z]²⁺ ·2X⁻ handeln,

wobei Z -N⁺R1R2R3 oder -R4R5N⁺-(CH₂)ₓR6C(=O)R7 ist, mit
- R1,R2,R3 =: Alkylreste mit 1 bis 22 Kohlenstoffatomen oder Alkenylreste mit 2 bis 22 Kohlenstoffatomen, wobei mindestens einer der Reste R1, R2, R3 mindestens 10 Kohlenstoffatome aufweist,
- R4,R5,R7 =: Alkylreste mit 1 bis 22 Kohlenstoffatomen oder Alkenylreste mit 2 bis 22 Kohlenstoffatomen,
- R6 = -: O- oder -NR8-Rest,
- R8 =: Alkyl- oder Hydroxyalkylrest mit 1 bis 4 Kohlenstoffatomen oder Wasserstoffrest,
- x =: 2 bis 4,
- M =: ein zweiwertiger Kohlenwasserstoffrest mit mindestens 4 Kohlenstoffatomen, der eine Hydroxylgruppe aufweist und der durch ein Sauerstoffatom unterbrochen sein kann,
- n =: eine Zahl von 0 bis 200,
- X⁻ =: anorganisches oder organisches Anion.
- M bevorzugt =: -(CH₂)₃OCH₂CH(OH)CH₂-, -(CH₂)₃OCH₂CH(CH₂OH)-, -(CH₂)₂-CH(OH)-CH₂--(CH₂)₂CH(CH₃)CH₂OH, -(CH₂)₃CH(OH)-CH₂-, -(CH₂)₃-CH(CH₃)CH₂OH

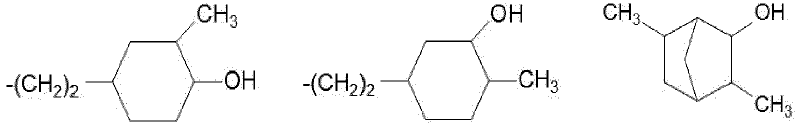

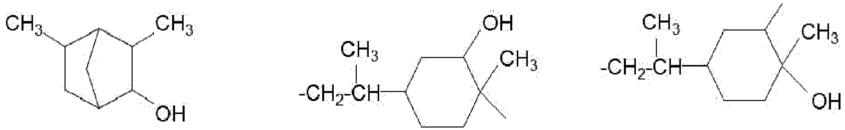

Die Herstellung dieser Verbindungen ist in EP-A-294642 beschrieben. Beispielhaft seien die Salze von Polysiloxanen mit zwei quarternären Ammoniumgruppen genannt, dargestellt durch die die statistischen Formeln
[Z-M-Si(CH₃)₂O[Si(CH₃)₂O]₁₀Si(CH₃)₂-M-Z]·X
Z = C₁₈H₃₇-^{⊕}N-(CH₃)₂, M= -CH₂-CH(OH)CH₂O(CH₂)₃-, X = 2 Cl⁻ oder Z= C₁₁H₂₃C(=O)-NH-(CH₂)₃-^{⊕}N-(CH₃)₂, M= CH₂-CH(OH)-(CH₂)₂-, X= 2 CH₃CO₂ oder Z= C₁₀H₂₁-^{⊕}N-(CH₃)₂; M= CH₂-CH(OH)-(CH₂)₂-, X= 2CH₃CO₂⁻.

Weiterhin kann es sich bei den kationischen Polysiloxanen um solche der allgemeinen Formel [MDₙ]₃T handeln. Dabei sind: M = XSiY₂O_{1/2} D = SiY₂O_{2/2}, T = SiZO_{3/2}, wobei
- X: sind gleiche oder verschiedene organische Reste, die quaternäre Ammoniumgruppen tragen,
- Y: sind gleiche oder verschiedene Reste aus der Gruppe Alkyl, Aryl, oder Alkaryl mit 1 bis 30 C-Atomen, vorzugsweise Methyl oder Phenyl, insbesondere Methyl,
- Z: sind gleiche oder verschiedene Reste aus der Gruppe Alkyl, Aryl, oder Alkaryl mit 1 bis 30 C-Atomen, vorzugsweise Methyl oder Phenyl,
- n: ist 2 bis 200, bevorzugt 3 bis 120, insbesondere 8 bis 80.

Es ist dem Fachmann geläufig, dass die Verbindungen in Form eines Gemisches mit einer im Wesentlichen durch statistische Gesetze geregelten Verteilung vorliegen. Die Einheit T ist im statistischen Mittel einmal in einer Polymerkette vorhanden. Es liegt jedoch eine Mischung von Molekülen vor, so dass ein gewisser Anteil der Molekülen keine oder mehrere T-Einheiten besitzen.

Geeignete Reste X sind zum Beispiel Gruppen mit dem Aufbau -R1-R2, worin
- R1: vorzugsweise gleiche oder verschiedene zweiwertige Reste sind, ausgewählt aus der Gruppe -(CH₂)₃OCH₂CH(OH)CH₂-, -(CH₂)₃OCH₂CH(CH₂OH)CH-, -(CH₂)ₓCH₂CH(OH)CH₂-, -(CH₂)ₓCH₂CH(CH₃)CH₂OH, _CH₂-CH(R3)-C(=O)-O-R4-CH(OH)-CH₂-, -CH₂-CH(R3)C(=O)-OR4-CH(CH₂OH)-; x = 2 bis 18. 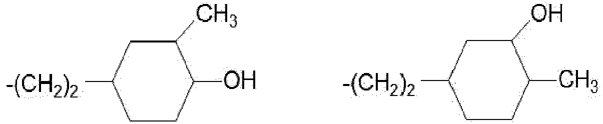 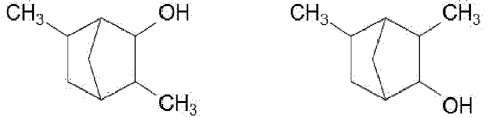
- R1: ist bevorzugt -(CH₂)₃OCH₂CH(OH)CH₂-,
- R2: ausgewählt ist aus der Gruppe bestehend aus 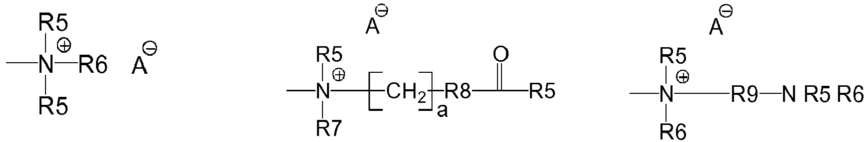 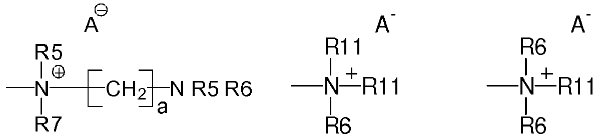
- a: 2 bis 18, vorzugsweise 3,
- A⁻: sind gleiche oder verschiedene Gegenionen zu den positiven Ladungen an den quaternären Stickstoffgruppen, ausgewählt aus anorganischen oder organischen Anionen der Säuren HA, sowie deren Derivate.
- R3: sind gleiche oder verschiedene Reste aus der Gruppe Wasserstoff oder Alkyl mit 1 bis 6 C-Atomen, bevorzugt Methyl,
- R4: sind gleiche oder verschiedene zweiwertige Kohlenwasserstoffreste, die gegebenenfalls Etherfunktionen enthalten, bevorzugt Methylen,
- R5, R6, R7: sind jeweils unabhängig voneinander Wasserstoff oder Alkylreste mit 1 bis 30 C-Atomen,
- R8: sind gleiche oder verschiedene Reste aus der Gruppe -O-; -NR10,
- R9: sind gleiche oder verschiedene gegebenenfalls verzweigte divalente Kohlenwasserstoffreste,
- R10: sind gleiche oder verschiedene Reste aus der Gruppe Wasserstoff oder Alkyl mit 1 bis 6 C-Atomen,
- R11: sind gleiche oder verschiedene Reste der allgemeinen Formel: 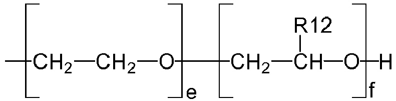
- R12: sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl,
- e: 0 bis 20, bevorzugt 0 bis 10, insbesondere 1 bis 3,
- f: 0 bis 20, bevorzugt 0 bis 10,
- e+f: >= 1.

Besonders bevorzugt handelt es sich bei den kationischen Polysiloxanen der allgemeinen Formel [MD_{n]3}T um solche mit mindestens drei quarternären Ammoniumgruppen. Beispielhaft seien die Salze von Polysiloxanen mit drei quarternären Ammoniumgruppen genannt, dargestellt durch die die statistischen Formeln 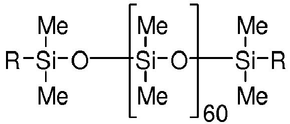 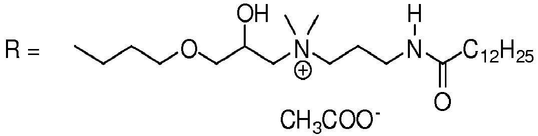 und

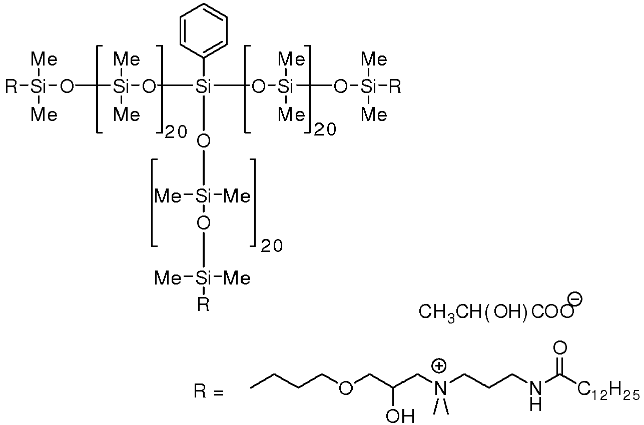

Sie stellen trübe, hochviskose Flüssigkeit dar. Dem Fachmann ist geläufig, dass die oben angegebenen Formeln idealisierte Strukturformeln darstellen. Im Produkt liegen zusätzlich lineare und höher verzweigte Strukturen vor. Die Herstellung der Verbindungen [MDₙ]₃T ist in EP-A-1887024 beschrieben.

Der Alkohol der erfindungsgemäßen Dispersion wird bevorzugt aus der Gruppe bestehend aus Ethanol, i-Propanol, n-Propanol, n-Butanol und/oder i-Butanol ausgewählt.

Das Treibmittel der erfindungsgemäßen Dispersion wird bevorzugt aus der Gruppe bestehend aus Propan, Propen, n-Butan, i-Butan, i-Buten, n-Pentan, Penten, i-Pentan, i-Penten, Methan, Ethane, Dimethylether, Stickstoff, Luft, Sauerstoff, 1,1,1,3-Tetrafluoroethan, Heptafluoro-n-propan, Perfluoroethan, Monochlorodifluoromethan und/oder 1,1-Difluoroethan gewählt. Besonders bevorzugt sind Propan, Butan und/oder i-Butan.

Ein weiterer Gegenstand der Erfindung ist die Verwendung der erfindungsgemäßen Zubereitung als Trockenshampoo.

### Beispiele

### Pulverförmige Zubereitung

### Beispiel 1

Es werden 47 g vollentsalztes Wasser, welches 0,5 Gew.-% Natriumbenzoat enthält und einen pH von < 5 aufweist, im Mischbehälter eines Küchenmixer (Gastroback Classic, Gastroback GmbH) vorgelegt. Anschließend werden 3 g AEROSIL^{®} R 812 S, Evonik Industries, und 50 g DRY FLO^{®} PC, mit Octenylbernsteinsäureanhydrid modifizierte und mit Aluminiumsulfat behandelte Stärke, Akzo Nobel, zugegeben. Der Mischbecher wird mit einem Deckel verschlossen. Anschließend wird 30 s lang bei Stufe 4 geschert. Es entsteht eine pulverförmige, frei fließende Zubereitung.

Die Beispiele 2-4 werden analog hergestellt. Die Zusammensetzung der Zubereitungen ist in Tabelle 1 wiedergegeben.

Vergleichsbeispiel: analog Beispiel 1, jedoch ohne AEROSIL^{®} R 812 S. Statt eines Pulvers wird hier nur eine viskose, cremeartige Dispersion erhalten.

**Tabelle 1: Pulverförmige Zubereitungen**

| **Beispiel^{a)}** | | **1** | **2** | **3** | **4** |
|---|---|---|---|---|---|
| DRY FLO® PC^{a)} | g | 50 | 50 | 67 | 33 |
| AEROSIL® R812S^{b}) | g | 3 | 4 | 3 | - |
| AEROSIL® R202^{b}) | g | - | - | - | 7 |
| Wasser | g | 47 | 46 | 30 | 60 |
| (DRY FLO® PC + AEROSIL®) / Wasser | g/g | 53:47 | 54:46 | 70:30 | 40:60 |
| AEROSIL® / DRY FLO® PC | g/g | 1:16,7 | 1:12,5 | 1:22,3 | 1:4,7 |

| | | | | | |
|---|---|---|---|---|---|
| a) INCI: Aluminum Starch Octenylsuccinate; Akzo Nobel; b) Evonik Industries | | | | | |

### Dispersionen (gemäß Erfindung)

Zur Herstellung der erfindungsgemäßen Dispersion wird die pulverförmige Zubereitung des Beispiels 1 oder 4 ausgewählt. Auch mit den pulverförmigen Zubereitungen der Beispiele 2 und 3 können erfindungsgemäße Dispersionen erhalten werden.

Die pulverförmige Zubereitung des Beispiels 1 oder 4 wird in einem Glasbehälter vorgelegt, die wässerige Ethanol-Lösung samt dem Polysiloxan wird hinzugefügt. Als letzter Schritt wird das Treibgas zugegeben. Man erhält die erfindungsgemäßen Dispersionen 1B, 1C, 1D und 4E.

### Dispersionen (Vergleich)

Die pulverförmigen Zubereitungen Beispiele 1 - 3 werden jeweils ohne Polysiloxan in einem Glasbehälter vorgelegt, die wässerige Ethanol-Lösung wird hinzugefügt. Als letzter Schritt wird das Treibgas zugegeben. Man erhält die Dispersionen 1A, 2A und 3A.

Die Zusammensetzung der Dispersionen ist in Tabelle 2 wiedergegeben.

Die erfindungsgemäßen Dispersionen 1B, 1C, 1D und 4E lassen sich gut auf das Haar aufsprühen. Als Substrat eignen sich besonders Echthaarbündel, z.B. von der Firma Kerling aus Euro-Naturhaar, mit 16cm freier Länge und einem Gewicht von 4g, welche durch Bleichen chemisch geschädigt wurden. Beim Auftreffen der Dispersion auf dem Haar bzw. beim Verreiben in den Haaren wird die Hülle zerstört und die wässerige Phase freigesetzt.

Die Bewertung der Wirkung der jeweiligen Dispersionen erfolgt mittels eines sensorischen Haarsträhnentest, der von speziell geschulten Sensorikteilnehmern durchgeführt wird. Die Teilnehmer verwenden hierzu einen professionellen Friseurkamm, der eine grob und eine fein gezahnte Hälfte hat. Zunächst werden die Haare mit der groben Seite des Kammes entwirrt. Die dafür nötige Kraft wird mittels eines festgelegten Bewertungssystems beurteilt. Anschließend wird die behandelte Haartresse mit der feinen Seite des Kammes gekämmt und die Trockenkämmbarkeit entsprechend des Bewertungssystems beurteilt. Es folgt eine sensorische Beurteilung des Trockengriffes der Haartresse. Durch fühlen mit den Fingerspitzen wird bewertet, wie sich die Haare anfühlen. Die Kriterien hierfür sind ebenfalls in dem Bewertungssystem festgelegt. Daraus ergibt sich eine klare Differenzierbarkeit in den Pflegeeigenschaften der unterschiedlichen Dispersionen.

Die festen Bestandteile aus den Dispersionen 1B, 1C, 1D können danach problemlos ausgekämmt werden. Bei der Dispersion 4E ist eine schlechtere Auskämmbarkeit festzustellen, die jedoch noch deutlich besser ist als bei den Vergleichsdispersionen 1A, 2A, 3A

Die Ergebnisse zeigen, dass gemäß der Erfindung für eine gute Auskämmbarkeit kationische Polysiloxane oder Polysiloxanen mit einer Aminogruppe vorhanden sein müssen. Diese Polysiloxane können jedoch nicht Bestandteil der pulverförmigen Zubereitung sein, da deren oberflächenmodifizierenden Eigenschaften die Bildung der wässerige Phase umhüllenden Partikel verhindert.

Die Erfindung umgeht diese Problematik, indem in der erfindungsgemäßen Dispersion die Polysiloxane und die pulverförmige Zubereitung nebeneinander vorliegen. Damit ist der Anteil der Polysiloxane nicht beschränkt und das erfrischende Gefühl auf der Kopfhaut beim Austritt des Wassers aus der pulverförmigen Zubereitung bleibt erhalten.

**Tabelle 2: Dispersionen enthaltend die pulverförmigen Zubereitungen**

| | | Vergleich | | | Gemäß Erfindung | | | |
|---|---|---|---|---|---|---|---|---|
| | | **1A** | **2A** | **3A** | **1B** | **1C** | **1D** | **4E** |
| Pulverförmige Zubereitung | g | 7,0 | 7,0 | 7,0 | 7,0 | 7,0 | 7,0 | 7,0 |
| Ethanol^{a)} | g | 3,7 | 3,7 | 3,7 | 3,7 | 3,7 | 3,7 | 3,7 |
| ABIL® Quat 3272^{b}) | g | 0 | 0 | 0 | 0,74 | 0 | 0 | 0 |
| ABIL® T Quat 60^{c)} | g | 0 | 0 | 0 | 0 | 0,57 | 0 | 0 |
| ABIL® Soft AF 200^{d)} | g | 0 | 0 | 0 | 0 | 0 | 0,37 | 0 |
| ABIL® Soft AF 100^{e)} | g | 0 | 0 | 0 | 0 | 0 | 0 | 0,30 |
| Drivosol 27D60^{f)} | g | 26,3 | 26,3 | 26,3 | 26,3 | 26,3 | 26,3 | 26,3 |
| Pulverförmige Zubereitung | Gew.-% | 18,9 | 18,9 | 18,9 | 18,5 | 18,6 | 18,7 | 18,7 |
| Drivosol 27D60 | Gew.-% | 71,1 | 71,1 | 71,1 | 69,7 | 70,0 | 70,4 | 70,4 |
| Ethanol | Gew.-% | 10 | 10 | 10 | 9,8 | 9,8 | 9,9 | 9,9 |
| ABIL® Quat 3272 | Gew.-% | 0 | 0 | 0 | 2,0 | 0 | 0 | 0 |
| ABIL® T Quat 60 | Gew.-% | 0 | 0 | 0 | 0 | 1,5 | 0 | 0 |
| ABIL® Soft AF 200 | Gew.-% | 0 | 0 | 0 | 0 | 0 | 1,0 | 0 |
| ABIL® Soft AF 100 | Gew.-% | 0 | 0 | 0 | 0 | 0 | 0 | 1,0 |

| | | | | | | | | |
|---|---|---|---|---|---|---|---|---|
| a) eingesetzt als 10 prozentige, wässerige Lösung; Angabe in Tabelle bezogen auf 100% EtOH; b-d)Evonik Industries; 1 Gew.-% bezogen auf die 10 prozentige, wässerige Lösung von EtOH; b) 50 prozentige Lösung; INCI: Quaternium-80; c) 65 prozentige Lösung; INCI: Silicone Quaternium-22; d) Aminopropyl Dimethicone; e) Methoxy PEG/PPG-7/3 Aminopropyl Dimethicone; f) Butan (mindestens 59,1%), Isobutan (mindestens 19%), Propan (mindestens 19,6%); jeweils Massenprozent; | | | | | | | | |

## Patentansprüche

1. Pulverförmige Zubereitung enthaltend Partikel, die Tröpfchen einer wässerigen Phase umhüllen, **dadurch gekennzeichnet, dass**
die Zubereitung hydrophobe Siliciumdioxidpartikel und eine hydrophobierte Stärke enthält, und wenigstens ein Teil der hydrophoben Siliciumdioxidpartikel Bestandteil der die Tröpfchen der wässerigen Phase umgebenden Hülle bildet.

2. Pulverförmige Zubereitung nach Anspruch 1, **dadurch gekennzeichnet, dass** die Hülle als weiteren Bestandteil wenigstens einen Teil der hydrophobierten Stärke enthält.

3. Pulverförmige Zubereitung nach den Ansprüchen 1 oder 2,
**dadurch gekennzeichnet, dass**
das Gewichtsverhältnis hydrophobe Siliciumdioxidpartikel zu hydrophobierter Stärke 1:50 bis 1:2 ist.

4. Pulverförmige Zubereitung nach den Ansprüchen 1 bis 3,
**dadurch gekennzeichnet, dass**
das Gewichtsverhältnis Summe hydrophobe Siliciumdioxidpartikel und hydrophobierte Stärke zur wässerigen Phase 70:30 bis 20:80 ist.

5. Verfahren zur Herstellung der pulverförmigen Zubereitung gemäß der Ansprüche 1 bis 4,
**dadurch gekennzeichnet, dass**
die Bestandteile der flüssigen Phase zunächst vorgelegt und gegebenenfalls homogenisiert werden, anschließend die hydrophoben Siliciumdioxidpartikel und die hydrophobierte Stärke zugegeben werden und die Mischung geschert wird.

6. Verfahren nach Anspruch 5, **dadurch gekennzeichnet, dass**
hydrophobe, pyrogen hergestellte Kieselsäure eingesetzt wird.

7. Verfahren nach den Ansprüchen 5 oder 6, **dadurch gekennzeichnet, dass**
die Methanolbenetzbarkeit der hydrophoben Siliciumdioxidpartikel wenigstens 50 ist.

8. Verfahren nach den Ansprüchen 5 bis 7, **dadurch gekennzeichnet, dass**
die hydrophobierte Stärke eine mit Octenylbernsteinsäureanhydrid oder Dodecenylbernsteinsäure modifizierte ist, die jeweils mit einem Salz des Aluminiums oder Calciums behandelt ist.

9. Dispersion enthaltend
die pulverförmige Zubereitung gemäß der Ansprüche 1 bis 4,
ein Silikonöl und/oder ein organomodifiziertes Siloxan, wobei das organomodifizierte Siloxan aus wenigstens einem Mitglied der Gruppe bestehend aus einem Silikonpolyether, einem kationischen Polysiloxan und einem mindestens eine Aminogruppe aufweisenden Polysiloxan ausgewählt ist, einen Alkohol und
ein Treibmittel.

10. Dispersion nach Anspruch 9, **dadurch gekennzeichnet, dass** sie 5-30 Gew.-% pulverförmige Zubereitung, 0,1 bis 5 Gew.-% eines Silikonöles und/oder eines organomodifizierten Siloxanes, 3 bis 40 Gew.-% Alkohol und 30 bis 90 Gew.-% Treibmittel enthält.

11. Dispersion nach den Ansprüchen 9 oder 10, **dadurch gekennzeichnet, dass** das kationische Polysiloxan ein Salz eines Polysiloxanes mit zwei oder drei quarternären Ammoniumgruppen ist.

12. Verwendung der Dispersion gemäß der Ansprüche 9 bis 11 als Trockenshampoo.
